# EUROPEAN PATENT APPLICATION

(11) **EP 4 119 100 A1**
(43) Date of publication of application: **18.01.2023**
(21) Application number: 21928365.2
(22) Date of filing: 17.08.2021
(51) Int. Cl.: A61F 2/36, A61F 2/46

(54) **BALL REMOVAL INSTRUMENT**

(30) Priority: 01.06.2021 JP 2021092314
(71) Applicant: Medmetalex Co., Ltd, Nishiyodogawa-ku Osaka-shi, Osaka 555-0012 (JP)
(72) Inventor: NAKAGAWA, Tomomi, Osaka-shi, Osaka 555-0012 (JP); AMINO, Hirokazu, Osaka-shi, Osaka 555-0012 (JP)
(74) Representative: VKK Patentanwälte PartG mbB
(86) International application number: PCT/JP2021/029987
(87) International publication number: WO 2022/254738

(57) **Abstract**

To provide a ball remover that can easily remove a caput ball from a neck taper of a stem of a prosthetic hip joint in a minimally invasive operation with a small incision region and that is durable.

A ball remover used in removing a caput ball fitted to a neck taper of a neck part projecting from the femur and arranged in a stem of a prosthetic hip joint comprising a body part inserted in the medullary cavity of the femur, the caput ball has a partially flat, notched lower end surface of ball and a taper hole into which the neck taper is fitted, the ball remover comprises a handle part to which an operator applies force to remove the caput ball and a bend part extending to the medial direction close to the median plane of a human body that is formed at a lower end of the handle part, the bend part is recessed to form a fork end so as to accommodate the neck part, a lower surface of a lower end part on the lateral side of the bend part far from the median plane abuts on an upper surface of a shoulder part at the boundary between the body part and the neck part, an upper surface of the fork end of the bend part abuts on the lower end surface of ball of the caput ball and/or a surface at a lower hemisphere region on the side of the lower end surface of ball below the equator parallel to the lower end surface of ball of the caput ball.

## Description

### [Technical Field]

The present invention relates to a ball remover to remove a caput ball fitted to a neck taper of a stem of a prosthetic hip joint.

### [Background Art]

For a patient in whom abnormality is found in the hip joint, operations such as hip replacement arthroplasty that replace a part of or the entire hip joint with a prosthetic hip joint is conducted. Components of the prosthetic hip joint on the side of the femur comprises a stem to be inserted into the medullary cavity of the femur and a caput ball with a ball shape fitted to a tip side of a neck part of the stem.

Operations for hip replacement arthroplasty include the primary operation that installs a prosthetic hip joint into the patient's hip joint for the first time and the revision operation that exchanges the already installed prosthetic hip joint with a new prosthetic hip joint partially or totally. In the primary operation, in order to replace the caput ball attached to the neck part once during the operation, the caput ball may need to be removed. In the revision operation, replacement of the stem is not necessary, but replacement of the components on the acetabulum side and the caput ball may be necessary, in that case, only the caput ball may need to be removed. The operation will be conducted with extreme caution not to damage the neck part or other when removing the caput ball. Especially, since a female taper part inside the caput ball and a male taper part at the tip of the neck part of the stem are very tightly tapered fitted, very strong force or shock is required to take off them and careful attention must be paid.

In the primary and revision operations in hip replacement arthroplasty, in order to install a prosthetic hip joint into a patient's body, the patient's body must be incised. In recent years, surgical instruments have been improved to minimize the range of the incision to reduce the burden of the patient. The invention according to Patent Document 1 is devised with the aim of providing a ball remover that can prevent deterioration of workability and operability even in the case that the range of the incision is restrained small.

### [Prior Art Documents]

### [Patent Documents]

[Patent Document 1] Japanese Utility Model Registration No. 3165299

### [Summary of the Invention]

### [Problems to be Solved by the Invention]

According to Fig.5 of Patent Document 1 (refer to Fig.13 attached herein), in the remover of this invention, by using an installation hole 303c formed on a femur component 303 to which an end part of a surgical instrument for installation and operation is attached when installing the femur component 303, a support member 211 is supported by the femur component 303 without being supported by making it directly abut on a surface of a neck part 303a. An energization member 212 is rotatably or pivotably supported by the support member 211, which is pivotably supported by the femur component 303. Applying impact force from a surgical instrument for hammering operation to a hammered part 225 of the energization member 212 makes an abutting part 224 in the opposite side of the hammered part 225 abut a caput ball 304 on its end surface or the like via the fulcrum to force the caput ball 304 in the direction of removal. Whereby the caput ball 304 is removed. The removal work of the caput ball 304 is conducted without damaging the neck part 303a of the femur component 303, and the ball remover 201 is compactly configured with the support member 211 supported with the installation hole 303c of the femur component 303 and the energization member 212 rotatably or pivotably supported by the support member 211, thereby miniaturizing the ball remover 201. Therefore, even in the case that the range of the incision is restrained small, deterioration of workability and operability can be prevented.

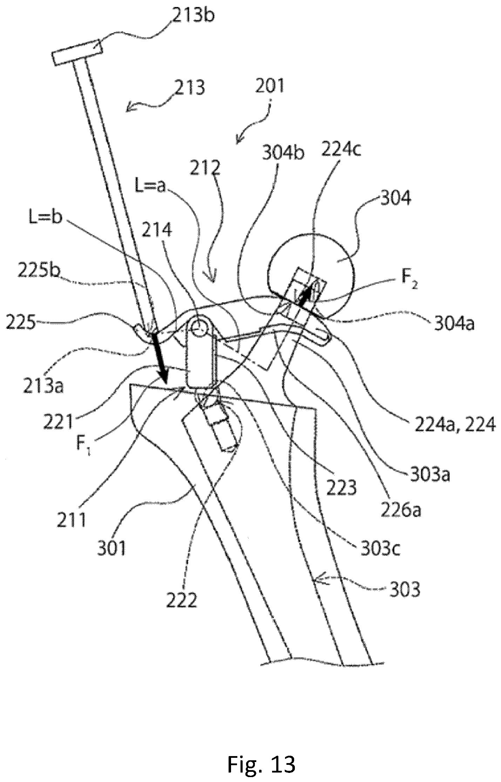

As shown in Fig.13, an end part of a hammering 213b is hammered, and the force applied from the hammered part 225 is F₁. The force applied to the caput ball 304 from the abutting part 224 of the energization member 212 is F₂ When the distance from the center of a hinge pin 214 to the hammered part is b and the vertical distance from the center of the hinge pin 214 in the direction of the force F₂ is a, the force F₂ is given by F₁ ×(b/a), therefore, the force F₂ is smaller by (b/a) than the applied force F₁, and the applying force F₁ has to be very large to remove the caput ball 304. Applying such stroke will not only raise a possibility of damaging the neck part 303a but also raise a risk of damaging the hinge pin 214.

The present invention is made considering such matters, and the purpose of the present invention is to provide a ball remover that can easily remove a caput ball from a neck taper of a stem of a prosthetic hip joint in a minimally invasive operation with a small incision region and that is durable.

### [Means to Solve the Problem]

The ball remover according to the present invention (1) made to solve the above problems is a ball remover used in removing a caput ball fitted to a neck taper of a neck part projecting from the femur and arranged in a stem of a prosthetic hip joint comprising a body part inserted in the medullary cavity of the femur, the caput ball has a partially flat, notched lower end surface of ball and a taper hole into which the neck taper is fitted, the ball remover comprises a handle part to which an operator applies force to remove the caput ball and a bend part extending to the medial direction close to the median plane of a human body that is formed at a lower end of the handle part. The bend part is characterized in that the bend part is recessed to form a fork end so as to accommodate the neck part, a lower surface of a lower end part on the lateral side of the bend part far from the median plane abuts on an upper surface of a shoulder part at the boundary between the body part and the neck part, an upper surface of the fork end of the bend part abuts on the lower end surface of ball of the caput ball and/or a surface at a lower hemisphere region on the side of the lower end surface of ball below the equator parallel to the lower end surface of ball of the caput ball, and when the handle part is pushed down toward the lateral direction, the caput ball receives the force from the upper surface of the fork end of the bend part to be removed from the neck taper with the fulcrum that is the abutting part between the lower surface of the lower end part on the lateral side of the bend part and the upper surface of the shoulder part.

The ball remover according to the present invention (1) is an application of a lever mechanism by the bell crank, a force larger than the force applied by the operator is applied to the caput ball form the upper surface of the fork end of the bend part, and thereby easily removing the caput ball. Further, a hinge pin is not used as the fulcrum as the invention described in Patent Document 1, therefore, the ball remover has a durable structure even when the force is applied to remove the caput ball.

The ball remover according to the present invention (2) is the ball remover according to the present invention (1), wherein a screw hole to insert the stem into the medullary cavity of the femur is arranged on the shoulder part of the stem, and a projection part with a substantially hemispherical shape projects on the lower surface of the lower end part on the lateral side of the bend part so as to abut with an opening of the screw hole.

In the ball remover according to the present invention (2), the projection part with a substantially hemispherical shape projects on the lower surface of the lower end part on the lateral side of the bend part so as to abut with the opening of the screw hole, therefore, force can be applied using this projection part as the fulcrum, and thereby resulting in more stabilized operability.

The ball remover according to the present invention (3) is the ball remover according to the present invention (2) further comprising a support plate arranged between the lower surface of the lower end part on the lateral side of the bend part and the upper surface of the shoulder part, and the support plate has a plate part abutting on the lower surface of the lower end part on the lateral side of the bend part instead of the upper surface of the shoulder part and an axis part projecting from a lower surface of the plate part to be inserted into the screw hole.

Since the ball remover according to the present invention (3) comprises the support plate arranged between the lower surface of the lower end part on the lateral side of the bend part and the upper surface of the shoulder part, even with a stem having no flat part on the upper surface of the shoulder part, operability can be more stabilized by providing the support plate.

The ball remover according to the present invention (4) is the ball remover according to any one of the present inventions (1) to (3) to which an accommodation plate arranged on the upper surface of the bend part can be additionally installed, characterized in that the accommodation plate has a prescribed thickness with a through hole through which the handle part penetrates, and has substantially the same shape as the shape of the fork end of the bend part at the plane view, and when the handle part is pushed down toward the lateral direction, the caput ball receives the force from the upper surface of the fork end of the accommodation plate to be removed from the neck taper.

Since the ball remover according to the present invention (4) further has the accommodation plate, even in the case where the length of the neck part in the longitudinal direction is long, the upper surface of the fork end of the bend part can be properly abutted on the lower end surface of ball and/or the surface at the lower hemisphere region on the side of the lower end surface of ball below the equator parallel to the lower end surface of ball of the caput ball.

The ball remover according to the present invention (5) is the ball remover according to any one of the present inventions (1) to (4), characterized in that a hammered part for hammering projecting from a surface on the lateral side of the handle part in the lateral direction is formed in the handle.

Since in the ball remover according to the present invention (5), the hammered part for hammering projecting from the surface on the lateral side of the handle part in the lateral direction is formed in the handle, the caput ball can be removed with impact by knocking the hammering part with a hammer, or the like. If it interferes with the insertion of the accommodation plate of the present invention (4), a through groove ranging with the through hole of the accommodation plate is formed such that the accommodation plate can be inserted from the handle part on the lower side of the hammered part, and the accommodation plate can be used despite the hammered part.

The ball remover according to the present invention (6) is a ball remover used in removing the caput ball fitted to the neck taper of the neck part projecting from the femur and arranged in the stem of the prosthetic hip joint comprising the body part inserted in the medullary cavity of the femur, the caput ball has the partially flat, notched lower end surface of ball and the taper hole into which the neck taper is fitted, the ball remover has a remover body comprising a handle part to which an operator applies force to remove the caput ball and a stopper part formed at a lower end of the handle part and extending to the medial direction close to the median plane of a human body, and a slide body that engages with the handle part and slides in the longitudinal direction of the handle part, characterized in that the slide body is recessed to form a fork end so as to accommodate the neck part, a lower surface of a lower end on the lateral side of the stopper part far from the median plane abuts on the upper surface of the shoulder part at the boundary between the body part and the neck part, an upper surface of the fork end of the slide body is allocated by the operator so as to abut on the lower end surface of ball of the caput ball and/or the surface at the lower hemisphere region on the side of the lower end surface of ball below the equator parallel to the lower end surface of ball of the caput ball, and when the handle part is pushed down toward the lateral direction, the caput ball receives the force from the upper surface of the fork end of the slide body to be removed from the neck taper with the fulcrum that is the abutting part between the lower surface of the lower end part on the lateral side of the stopper part and the upper surface of the shoulder part.

The ball remover according to the present invention (6) has the remover body and the slide body, and has a structure where the lower surface of the lower end part of the stopper part of the remover body abuts on the upper surface of the shoulder part, the upper surface of the fork end of the slide body is allocated by the operator so as to abut on the lower end surface of ball and/or the surface at the lower hemisphere region on the side of the lower end surface of ball below the equator parallel to the lower end surface of ball of the caput ball, and when the handle part is pushed down toward the lateral direction, the caput ball receives the force from the upper surface of the fork end of the slide body to be removed from the neck taper with the fulcrum that is the abutting part between the lower surface of the lower end part on the lateral side of the stopper part and the upper surface of the shoulder part. With this structure, sliding the slide body makes handling possible even when the length of the neck part (neck length) of the stem changes. In addition, the structure according to the present invention (2), the support plate according the present invention (3), and the hammered part according to the present invention (5) can be added to the present invention (6).

### [Effect of the invention]

According to the present inventions, a ball remover that can easily remove a caput ball from a neck taper of a stem of a hip joint in a minimally invasive operation with a small incision region and that is durable can be provided.

### [Brief description of the drawings]

[Fig.1] A schematic diagram illustrating a situation where a stem of a prosthetic hip joint to which a caput ball is fitted is inserted into the femur.
[Fig.2] A perspective view illustrating a ball remover according to an aspect of Embodiment 1.
[Fig.3] A schematic diagram illustrating a situation where the ball remover according to an aspect of Embodiment 1 is used.
[Fig.4] A schematic diagram illustrating a situation where the caput ball comes off from the neck taper changing over from the situation shown in Fig.3.
[Fig.5] Schematic diagrams illustrating a ball remover according to an aspect of Embodiment 2 in which (A) shows a longitudinal section view around the bend part, (B) shows a front view, (C) shows a side view, (D) shows a rear side view, and (E) schematically shows a situation where the ball remover is actually used.
[Fig.6] Diagrams illustrating the ball remover according to an aspect of Embodiment 2 in which (A) is a perspective view illustrating the whole and (B) is an enlarged perspective view of the vicinity of the bend part.
[Fig.7] Schematic diagrams illustrating a ball remover comprising a support plate according to an aspect of Embodiment 3 in which (A) is a schematic diagram illustrating a situation where the ball remover is used and (B) is a schematic diagram of the stem to which the support plate is arranged when seeing from the rear side.
[Fig.8] Diagrams illustrating a ball remover comprising an accommodation plate according to an aspect of Embodiment 4 in which (A) shows a top view of the accommodation plate, (B) shows a cross section view of A-A, (C) shows a partial top view illustrating a situation where a piece of the accommodation plate is attached, and (D) shows a cross section view of (C).
[Fig.9] A schematic diagram illustrating a ball remover in which a hammered part is formed according to an aspect of Embodiment 5 and showing a situation where the caput ball is actually removed by hammering the hammered part using a hammer.
[Fig.10] Schematic diagrams illustrating a ball remover according to an aspect of Embodiment 5 in which (A) shows a situation where a slide body abuts on a stopper part, (B) shows a situation where the slide body is slid and moved up, and (C) shows a situation where a force is applied to the handle part to remove the caput ball from the situation shown in (B).
[Fig.11] A top view (A) illustrating the slide body and a partially cross sectioned side view (B) illustrating the slide body.
[Fig.12] Diagrams illustrating various engaging conditions between the handle part and the slide body.
[Fig.13] A diagram illustrating a situation where the caput ball is being removed using the ball remover described in Patent Document 1.

### [Description of Embodiments]

Embodiments of the present invention will be described hereinafter based on the drawings. Please note that the embodiments below are essentially preferable examples, and do not intend to restrict the scope of the present invention, its applicable materials or its applications.

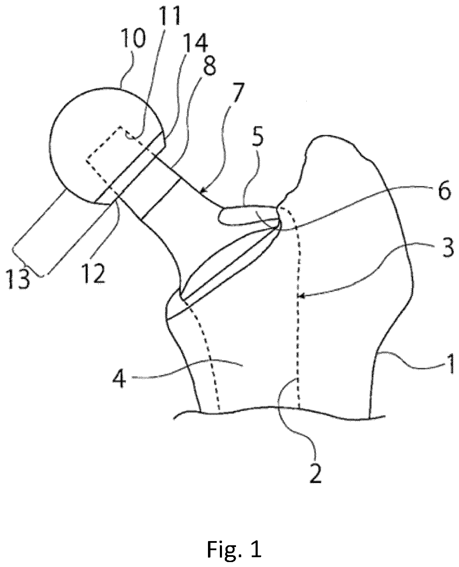

Fig.1 is a schematic diagram illustrating a situation where a stem of a prosthetic hip joint to which a caput ball is fitted is inserted into the femur. The body part 4 of the stem 3 is inserted into the medullary cavity 2 of the femur 1, the caput ball 10 having the taper hole 11 is fitted to the neck taper 8 of the neck part 7 projecting on the upper part of the femur 1. The surface at the lower end of the caput ball 10 is the lower end surface of ball 12, the region indicated by the reference numerical 13 is a region called a lower hemisphere region. The shoulder part 5 is located between the body part 4 of the stem 3 and the neck part 7, in the stem 3 shown in Fig.1, the upper surface of the shoulder part 6 that is an upper surface of the shoulder part 5 is a plane.

### <Embodiment 1>

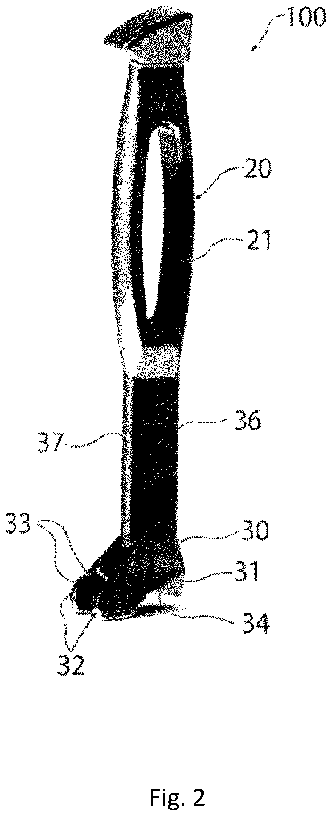

Figs. 2 to 4 relate to the ball remover according to Embodiment 1. Fig.2 is a perspective view of the ball remover according to an aspect of Embodiment 1, the ball remover 100 comprises the handle part 20 comprising the hollow hole 21 and the bend part 30 extending to the medial direction close to the median plane of a human body that is formed at the lower end of the handle part 20. The bend part 30 is recessed to form the fork end 32 so as to accommodate the neck part 7. The upper surface 33 of the fork end 32 is the region that is the load part applying force to the caput ball 10. The lateral side of the lower surface 34 of the lower end part 31 of the bend part 30 is the part that is the fulcrum. The surface 36 on the lateral side and the surface 37 on the medial side of the handle part 20 are also shown.

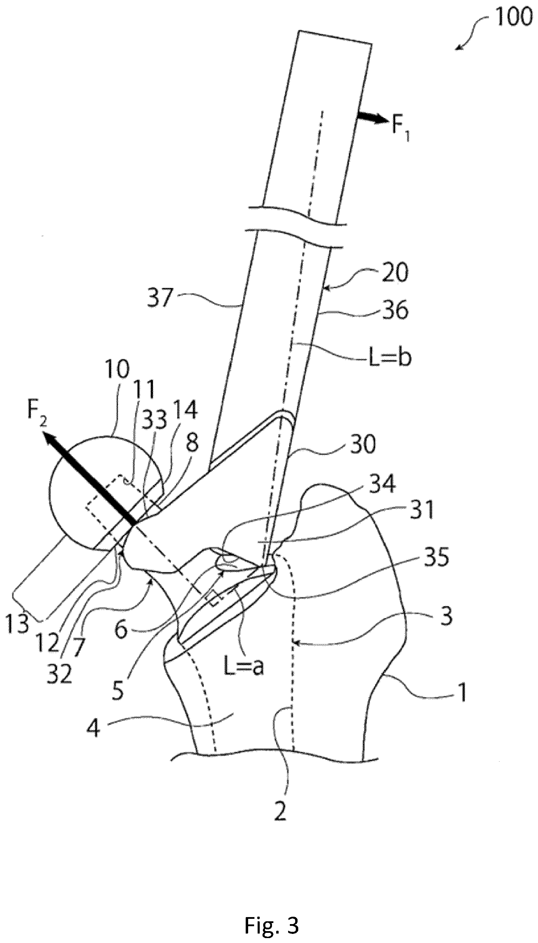

Fig.3 shows a situation where the ball remover 100 according to Embodiment 1 is allocated to start removing the ball 10. The operator grips the handle 20 to make the lower surface 34 of the lower end part abut on the upper surface of the shoulder part 6 to apply the force F₁. The abutting part 35 is the fulcrum for this operation. The upper surface 33 of the fork end abuts on the lower end part of ball 12, and this part is the load. Further, the outer taper surface 14 is formed between the lower end part of ball 12 and the outer spherical surface of the ball, so the load may be located on this surface.

Comparing the force F₁ applied by the operator with the forceF₂ that the ball 10 receives from the ball remover 100 shown in Fig.3, when the distance from the abutting part 35 (fulcrum) to where the force F₁ is applied is b and the vertical distance between the abutting part 35 (fulcrum) and the vector of the force F₂ is a, it gives F₂ = F₁ × b/a, therefore, the force F₂ to remove the caput ball 10 is a coefficient b/a times the applying force F₁, and a small force can be made into a large load force. This principle is a lever mechanism by the bell crank, and the present invention applies this mechanism.

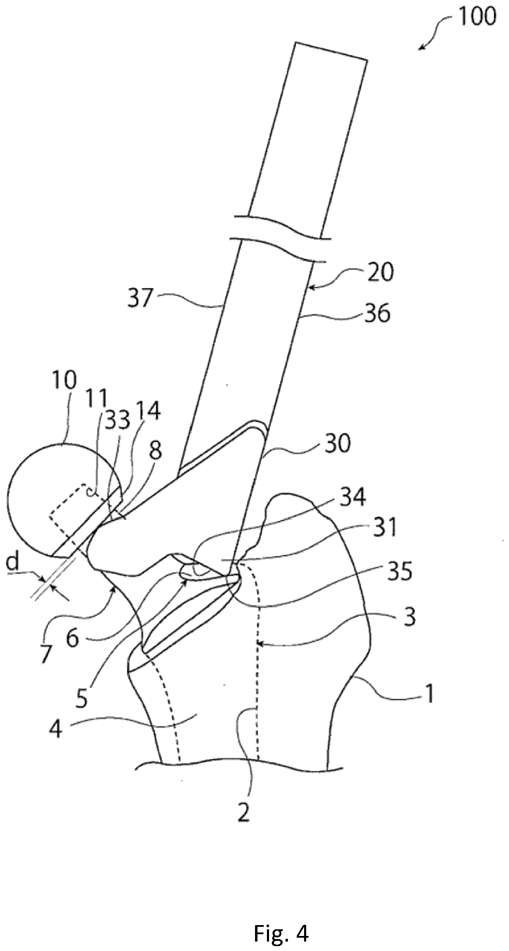

Fig.4 is a schematic diagram illustrating a situation where the caput ball comes off from the neck taper changing over from the situation shown in Fig.3, but in the situation shown in Fig.4, the caput ball 10 is shifted to the axis direction of the neck taper 8 by the distance d compared to the situation shown in Fig.3, resulting in a situation where the caput ball 10 is not fitted to the neck taper 8.

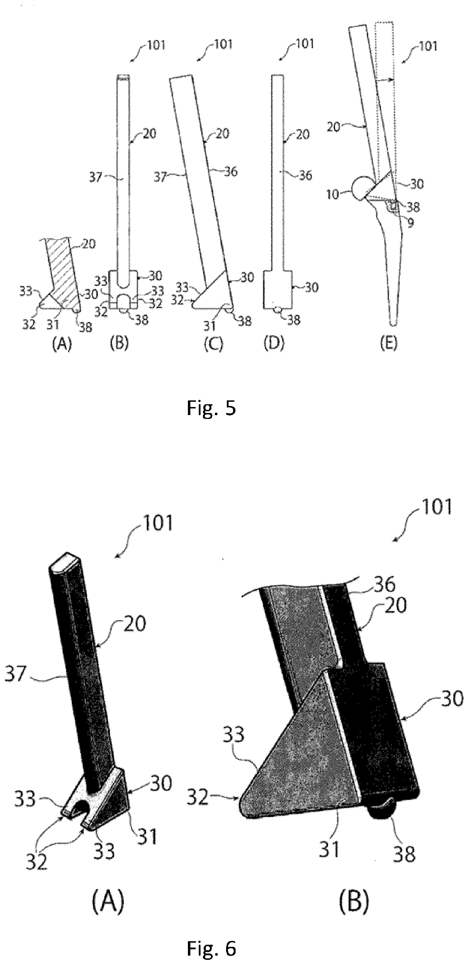

Fig.5 shows the ball remover 101 according to an aspect of Embodiment 2. The same reference numerical is given to the parts that serve the same function as Embodiment 1. (A) is a longitudinal cross section view around the bend part, the ball remover 101 is characterized in that the projection part 38 is formed. (B) shows a front view, (C) shows a side view, (D) shows a rear side view, and (E) schematically shows a situation where the ball remover is actually used. Looking at (E), the projection part 38 enters into the screw hole 9, this projection part 38 has a substantially hemisphere shape, so that it stably moves according to the movement of the handle part 20.

Fig.6 shows the ball remover according to an aspect of Embodiment 2, (A) is a perspective view illustrating the whole and (B) is an enlarged perspective view of the vicinity of the bend part.

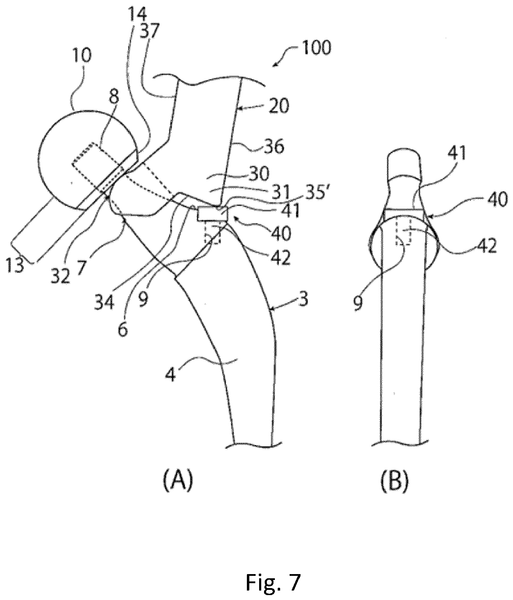

Fig.7 shows the ball remover comprising the support plate according to an aspect of Embodiment 3, (A) is a schematic diagram illustrating a situation where the ball remover is used and (B) is a schematic diagram of the stem to which the support plate is arranged when seeing from the rear side. In the type of the stem 3 shown in Fig.7, the upper surface of the shoulder part 6 is not planar but curved, therefore, the stability upon using the ball remover 100 is a little inferior. To improve this stability, the support plate 40 is intervened to stabilize. The support plate 40 comprises the plate part 41, the axis part 42 is formed on the central part of its lower surface, and the axis part 42 is inserted into the screw hole 9. In this figure, the fulcrum is the abutting part 35'.

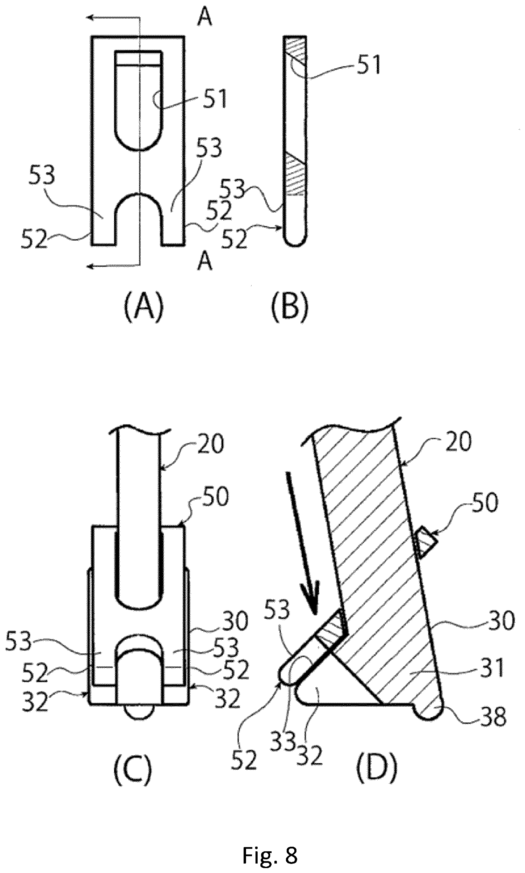

Fig.8 shows the ball remover comprising the accommodation plate according to an aspect of Embodiment 4, (A) shows a top view of the accommodation plate 50, (B) shows a cross section view of A-A, (C) shows a partial top view illustrating a situation where a piece of the accommodation plate 50 is attached, and (D) shows a cross section view of (C). In the accommodation plate 50, the through hole 51 is formed and the accommodation plate fork end 52 is formed. The shape of the accommodation plate fork end 52 has substantially the same shape as the fork end 32 at the plane view. The upper surface of the accommodation plate fork end 53 on the upper surface of the accommodation plate fork end 52 abuts on the caput ball 10. As shown in the arrow (D), the accommodation plate 50 is shifted downward along the handle part 20 and installed. As necessary, multiple plates can be installed. By using the accommodation plate 50, even in the case where the length of the neck part 7 in the longitudinal direction is long, the upper surface of the fork end 33 of the bend part 30 can be properly abutted on the lower end surface of ball 12 and/or the surface at the lower hemisphere region 13 on the side of the lower end surface of ball 12 below the equator parallel to the lower end surface of ball 12 of the caput ball 10. In Fig.8, although the periphery of the through hole 51 is surrounded, depending on the shape of the handle part 20, it is possible that a notched groove is partially formed in the periphery of the through hole 51, and a region with a smaller longitudinal cross section is made on the handle part 20, so that the region passes through the notched groove to install the accommodation plate 50.

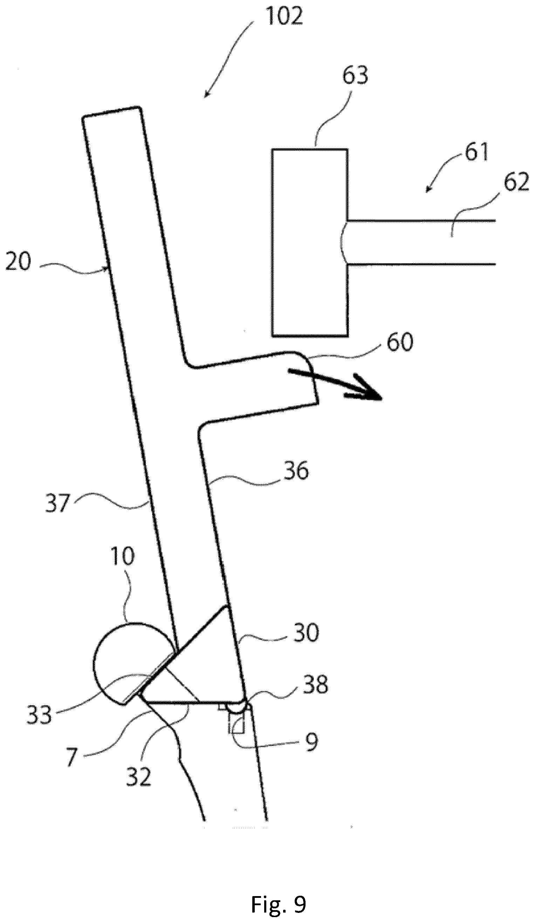

Fig.9 shows the ball remover 102 in which the hammered part 60 is formed according to an aspect of Embodiment 5 and a situation where the caput ball 10 is actually removed by hammering the hammered part 60 using the hammer 61. The operator grips the hammer handle 62, then strikes the hammered part 60 with the hammering part 63. A significantly larger force can be instantly applied compared with the net load power by striking, therefore the force to take off the caput ball 10 can be smaller. When installing the accommodation plate 50 to this type of the ball remover 102, it is required that a notched groove is partially formed in the periphery of the through hole 51, and a region with a smaller longitudinal cross section is made on the handle part 20, so that the region passes through the notched groove to install the accommodation plate 50.

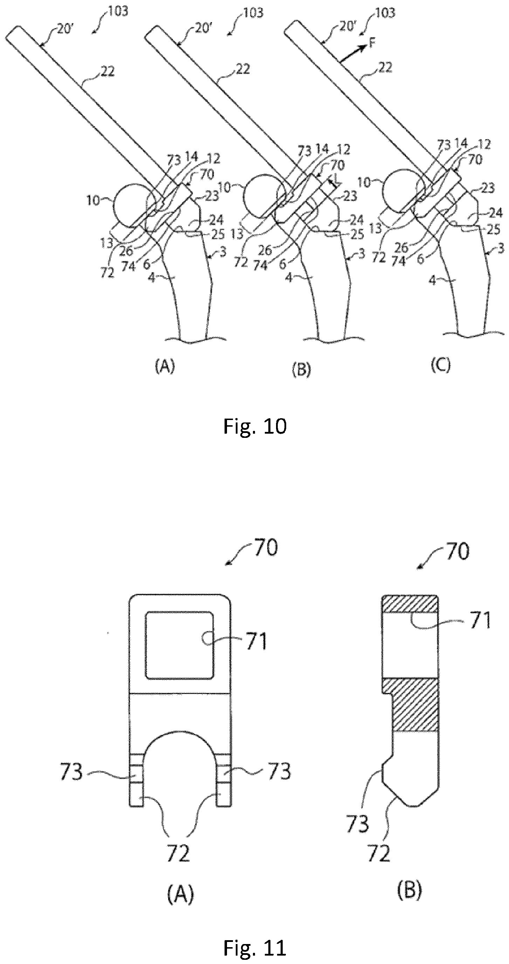

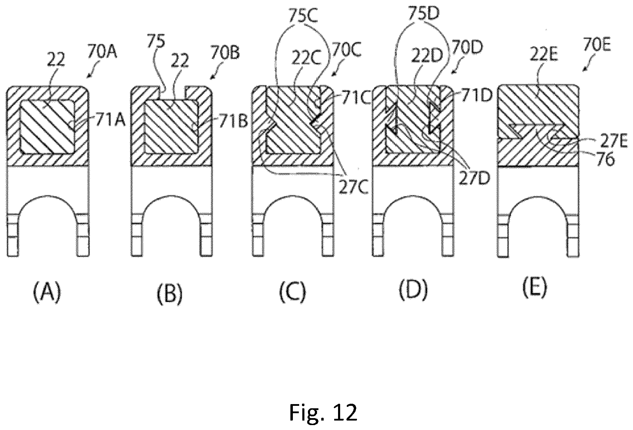

Fig.10 shows the ball remover according to an aspect of Embodiment 5. (A) shows a situation where the slide body 70 abuts on the stopper part 23. This ball remover 103 has two parts of the remover body 20' and the slide body 70. The remover body 20' comprises the handle part 22 and the stopper part 23. The surface on the lower side of the lower end part 24 of the stopper 23 is the lower surface of the lower end part 25, which abuts on the upper surface of the shoulder part 6 of the body part 4 of the stem 3. The through hole 71 through which the handle part 22 penetrates is made on the slide body 70 shown in Fig.11 (refer to Fig.11), and the penetration of the handle part 22 through the through hole 71 makes the remover body 20' engage with the slid body 70. As for the engaging method other than the relation between the through hole and the bar, a groove may be formed on the handle part 22 in the longitudinal direction, and a projection to fall into the groove may be formed on the slide body 70 for engagement. Or a groove may be formed on the slide body 70 and a projection to fall into the groove may be formed on the handle part 22 in the longitudinal direction for engagement. Specific examples of various engagement conditions will be shown in Fig.12 below. The aspects shown in Fig. 12 are merely examples, and any aspect using the technical concept of the present invention will be available.

In Fig10 (A), since the upper surface of the stopper part 26 abuts on the lower surface of the slide body 74, and the upper surface of the slide body fork end 73 abuts on the lower end surface of ball 12, when the handle part 22 is pushed toward the lateral direction, the caput ball 10 receives the force from the upper surface of the fork end 73 of the slide body 70 to be removed from the neck taper 8 (refer to Fig.1) with the fulcrum that is the abutting part between the lower surface 25 of the lower end part 24 on the lateral side of the stopper part 23 and the upper surface of the shoulder part 6.

In Fig.10(B), compared with Fig.10(A), the neck length of the neck part 7 (refer to Fig.1) is longer, thus the operator allocates the upper surface of the slide body fork end 73 to the lower end surface of ball 12 by shifting the slide body 70. The arrow indicates the length L shifted by the operator.

Fig.10(C) shows a situation where the handle part 22 is pushed down by applying the force F in the direction of the lateral side with the upper surface of the slide body fork end 73 being allocated to the lower end surface of ball 12 by shifting the slide body 70. Once pushed down by the force F, the caput ball 10 receives the force from the upper surface of the fork end 73 of the slide body 70 to be removed from the neck taper 8 with the fulcrum that is the abutting part between the lower surface 25 of the lower end part 24 on the lateral side of the stopper part 23 and the upper surface of the shoulder part 6. At the time, the slide body 70 will not shift downward due to the engagement friction with the handle part 22.

In Fig.11, (A) shows a top view of the slide body 70 and (B) shows a partially cross sectioned side view of it. The through hole 71 through which the handle part 22 penetrates, the slide body fork end 72, the upper surface of the slide body fork end 73, and the lower surface of the slide body 74 are formed.

In Fig.12, examples of the engagement condition between the handle part 22 and the slide body 70 are merely shown, and the engagement condition according to the present invention shall not be limited to Fig. 12. Any structure in which a groove or the like to slide is formed on either the handle part 22 or the slide body 70 and the other part to fall into the groove or the like and slide is formed on the other is sufficient.

In Fig.12(A), the transverse section of the handle part 22 is substantially square, and the slide body 70A has the slide body through hole 71A with a substantially square shape on the transverse section. This substantially square shape may take various shapes including rectangular, polygon, round, ellipse, star shape, etc.

In Fig.12(B), the transverse section of the handle part 22 is substantially square, and the slide body recess 71B on which the slide body notch 75 is formed is formed in the slide body 70B on the transverse section. This substantially square shape of the handle part 22 on the transverse section may take various shapes including rectangular, polygon, round, ellipse, star shape, etc., and the slide body 70 may also take various shapes in accordance with the shape. In Fig.12(B), the slide body notch 75 is disposed in a single area on the lateral side, but it is also possible to dispose it in another position.

In Fig.12(C), the triangle handle part recesses 27C are formed right and left in the handle part 22C, the slide body recess 71C is formed in the slide body 70C, and the slide body recess projections 75C are formed in the slide body recess 71C on the transverse section. The handle part recesses 27C engage with the slide body recess projections 75C, accordingly the slide body 70C slides on the handle 22. The cross-sectional shape of the triangle handle part recesses 27C and the slide body recess projections 75C on the transverse section is not limited to triangle as long as it has convexo-concave relation, and it can take various shapes including rectangular, polygon, round, ellipse, star shape, etc. Further, the number of the engaging positions does not need to be two, and one is sufficient.

In Fig.12(D), the handle part recesses 27D with a groove shape are formed right and left in the handle part 22, the slide body recess 71D is formed in the slide body 70D, and the slide body recess projections 75D are formed in the slide body recess 71D on the transverse section. The handle part recesses 27D engage with the slide body recess projections 75D, accordingly the slide body 70D slides on the handle 22D. The cross-sectional shape of the handle part recesses 27D and the slide body recess projections 75D with a dovetail groove shape on the transverse section is not limited to the dovetail groove shape, and it can take various shapes. Further, the number of the engaging positions does not need to be two, and one is sufficient.

In Fig.12(E), the handle part recess 27E with a groove shape is formed in the handle part 22E on the medial side, and the slide body projection 76 is formed in the slide body 70E on the transverse section. The handle part recess 27E engages with the slide body projection 76, accordingly the slide body 70E slides on the handle part 22E. The cross-sectional shape of the handle part recess 27E and the slide body projection 76 with a dovetail groove shape on the transverse section is not limited to the dovetail groove shape, and it can take various shapes. Further, the number of the engaging position does not need to be one, and it may be multiple.

The present international application claims the priority based on Japanese Patent Application 2021-92314 filed on June 1, 2021, and the entire contents of Japanese Patent Application 2021-92314 are hereby incorporated into the present international application.

### [Industrial Applicability]

As described above, the ball remover according to the present invention can easily remove the caput ball from the neck taper of the stem of the hip joint in a minimally invasive operation with a small incision region and is durable, therefore it can be preferably used in hip joint operations.

### [Description of the Reference Numerals]

1 Femur
2 Medullary cavity
3 Stem
4 Body part
5 Shoulder part
6 Upper surface of shoulder part
7 Neck part
8 Neck taper
9 Screw hole
10 Caput ball
11 Taper hole
12 Lower end surface of ball
13 Lower hemisphere region
14 Outer taper surface
20, 22 Handle part
20' Remover body
21 Hollow hole
22, 22C, 22D, 22E Handle part
23 Stopper part
24 Lower end part
25 Lower surface of lower end part
26 Upper surface of stopper part
27C, 27D, 27E Handle part recess
30 Bend part
31 Lower end part
32 Fork end
33 Upper surface of fork end
34 Lower surface of lower end part
35, 35' Abutting part (fulcrum)
36 Surface on lateral side
37 Surface on medial side
38 Projection part
40 Support plate
41 Plate part
42 Axis part
50 Accommodation plate
51 Through hole
52 Accommodation plate fork end
53 Upper surface of accommodation plate fork end
60 Hammered part
61 Hammer
62 Hammer handle
63 Hammering part
70, 70A, 70B, 70C, 70D, 70E Slide body
71 Through hole
71A Slide body through hole
71B, 71C, 71D Slide body recess
72 Slide body fork end
73 Upper surface of slide body fork end
74 Lower surface of slide body
75 Slide body notch
75C Slide body recess projection
76 Slide body projection
100, 101, 102, 103 Ball remover

## Claims

1. A ball remover used in removing a caput ball fitted to a neck taper of a neck part projecting from the femur and arranged in a stem of a prosthetic hip joint comprising a body part inserted in the medullary cavity of the femur, the caput ball having a partially flat, notched lower end surface of ball, and a taper hole into which the neck taper is fitted, the ball remover comprising:
a handle part to which an operator applies force to remove the caput ball; and
a bend part extending to the medial direction close to the median plane of a human body that is formed at a lower end of the handle part, **characterized in that**
the bend part is recessed to form a fork end so as to accommodate the neck part,
a lower surface of a lower end part on the lateral side of the bend part far from the median plane abuts on an upper surface of a shoulder part at the boundary between the body part and the neck part,
an upper surface of the fork end of the bend part abuts on the lower end surface of the caput ball and/or a surface at a lower hemisphere region on the side of the lower end surface of ball below the equator parallel to the lower end surface of the caput ball, and
when the handle part is pushed down toward the lateral direction, the caput ball receives the force from the upper surface of the fork end of the bend part to be removed from the neck taper with the fulcrum that is the abutting part between the lower surface of the lower end part on the lateral side of the bend part and the upper surface of the shoulder part.

2. The ball remover according to Claim 1, wherein a screw hole to insert the stem into the medullary cavity of the femur is arranged on the shoulder part of the stem, and a projection part with a substantially hemispherical shape projects on the lower surface of the lower end part on the lateral side of the bend part so as to abut with an opening of the screw hole.

3. The ball remover according to Claim 2 further comprising a support plate arranged between the lower surface of the lower end part on the lateral side of the bend part and the upper surface of the shoulder part, wherein the support plate has a plate part abutting on the lower surface of the lower end part on the lateral side of the bend part instead of the upper surface of the shoulder part and an axis part projecting from a lower surface of the plate part to be inserted into the screw hole.

4. The ball remover according to any one of Claims 1 to 3 to which an accommodation plate arranged on the upper surface of the bend part can be additionally installed, **characterized in that** the accommodation plate has a prescribed thickness with a through hole through which the handle part penetrates and has substantially the same shape as the shape of the fork end of the bend part at the plane view, and when the handle part is pushed down toward the lateral direction, the caput ball receives the force from the upper surface of the fork end of the accommodation plate to be removed from the neck taper.

5. The ball remover according to any one of Claims 1 to 4, **characterized in that** a hammered part for hammering projecting from a surface on the lateral side of the handle part in the lateral direction is formed in the handle.

6. A ball remover used in removing a caput ball fitted to a neck taper of a neck part projecting from the femur and arranged in a stem of a prosthetic hip joint comprising a body part inserted in the medullary cavity of the femur, the caput ball having a partially flat, notched lower end surface of ball, and a taper hole into which the neck taper is fitted, the ball remover having:
a remover body comprising a handle part to which an operator applies force to remove the caput ball and a stopper part formed at a lower end of the handle part and extending to the medial direction close to the median plane of a human body; and
a slide body engaging with the handle part and sliding in the longitudinal direction of the handle part, **characterized in that**
the slide body is recessed to form a fork end so as to accommodate the neck part,
a lower surface of a lower end on the lateral side of the stopper part far from the median plane abuts on an upper surface of a shoulder part at the boundary between the body part and the neck part,
an upper surface of the fork end of the slide body is allocated by the operator so as to abut on the lower end surface of the caput ball and/or the surface at the lower hemisphere region on the side of the lower end surface of ball below the equator parallel to the lower end surface of the caput ball,
and when the handle part is pushed down toward the lateral direction, the caput ball receives the force from the upper surface of the fork end of the slide body to be removed from the neck taper with the fulcrum that is the abutting part between the lower surface of the lower end part on the lateral side of the stopper part and the upper surface of the shoulder part.
